# EUROPEAN PATENT APPLICATION

(11) **EP 4 173 580 A1**
(43) Date of publication of application: **03.05.2023**
(21) Application number: 21207076.7
(22) Date of filing: 09.11.2021
(51) Int. Cl.: A61B 17/34, A61B 18/14, A61B 34/10, G06N 3/02, G06N 20/00, A61B 34/20, A61B 90/00

(54) **DETERMINATION APPARATUS FOR DETERMINING A VIRTUAL POSITION OF A VIRTUAL GUIDANCE DEVICE**

(30) Priority: 27.10.2021 US 202163272372 P
(71) Applicant: Koninklijke Philips N.V., 5656 AG Eindhoven (NL)
(72) Inventor: HAUTVAST, Guillaume Leopold Theodorus Frederik, Eindhoven (NL); THOMPSON, Scott Frederic, Eindhoven (NL)
(74) Representative: Philips Intellectual Property & Standards

(57) **Abstract**

The invention relates to a determination apparatus for determining a virtual position of a virtual guidance device 119 like a virtual transperineal grid template for guiding a virtual ablation applicator 112 like a virtual ablation needle. A model is trained based on training data sets including real positions of real guidance devices used for guiding real ablation applicators during real ablation procedures and images of ablation targets and/or parameters extracted from the images. The parameters are, for instance, spatial parameters like the center of the respective ablation target. The model then is used for determining the virtual position of the virtual guidance device based on an image of the ablation target, which might be a pre-operative CT or MR image, and the model. This allows to provide a virtual position, which really is achievable by a real guidance device, wherein this allows for an improved ablation planning.

## Description

### FIELD OF THE INVENTION

The invention relates to a determination apparatus, a determination method and a determination computer program for determining a virtual position of a virtual guidance device for guiding a virtual ablation applicator.

### BACKGROUND OF THE INVENTION

Ablation applicators are used in ablation procedures like a percutaneous thermal ablation procedure. A percutaneous thermal ablation procedure uses one or several applicators, which are generally shaped as needles, for applying ablation energy to an ablation target like a tumor. The ablation energy can be provided by using, for example, radiofrequency (RF), microwave (MW), high-intensity focused ultrasound (HIFU), focal laser ablation (FLA), irreversible electroporation (IRE) or cryoablation.

The applicators are placed inside or near the ablation target with the help of image guidance. For instance, a physician places the applicators while inspecting a real-time ultrasound image of the ablation target and the surrounding tissue, which might be fused with a pre-operative radiology image like a computed tomography (CT) or magnetic resonance (MR) images.

Before carrying out the ablation procedure, the ablation procedure can be planned by using a pre-planning application. The planning is based on a pre-operative radiology image and can be used to answer questions like whether the patient is eligible for percutaneous thermal ablation and which type of ablation energy and how many applicators should be used. The pre-planning often is not very accurate which then requires to determine a new plan during the actual ablation procedure.

### SUMMARY OF THE INVENTION

It is an object of the present invention to provide a determination apparatus, a determination method and a determination computer program which allow for a determination of a virtual position of a virtual guidance device for guiding a virtual ablation applicator such that a planning of an ablation procedure, which is based on the determined virtual position of the virtual guidance device, can be improved.

In a first aspect of the present invention a determination apparatus for determining a virtual position of a virtual guidance device for guiding a virtual ablation applicator is presented, wherein the determination apparatus comprises:
- a model providing unit configured to provide a model that is adapted to output a virtual position of a virtual guidance device, if the model is provided, as an input, with an image of an ablation target or with a parameter extracted from the image of the ablation target, wherein the model providing unit is configured to train the model based on training data sets, wherein each training data set includes a) a real position of a real guidance device that has been used for guiding a real ablation applicator during a real ablation procedure for ablating a respective ablation target and b) an image of the respective ablation target and/or a parameter extracted from the image,
- an image providing unit configured to provide an image of the ablation target, and
- a virtual position determination unit configured to determine the virtual position of the virtual guidance device based on the provided image of the ablation target and the provided model.

It has been found that the value of ablation plans, which could also be named pre-plans and which are generated to prepare for an ablation procedure, is often very limited because assumed positions of guidance devices such as transperineal grid templates deviate too much from realistically achievable positions due to differences in subject positioning or, for example, a placement of endorectal probes if they are used, or differences in rectal filling if required. It is hence often required to determine a new plan during the actual ablation procedure. In order to overcome this drawback, according to the present invention, a model is provided, which is adapted to output a virtual position of a virtual guidance device, if the model is provided, as an input, with an image of the ablation target or with a parameter extracted from the image of the ablation target, wherein the model is trained based on training data sets, wherein each training data set includes a) a real position of a real guidance device that has been used for guiding a real ablation applicator during a real ablation procedure for ablating a respective ablation target and b) an image of the respective ablation target and/or a parameter extracted from the image. Since the virtual position of the virtual guidance device is determined based on, inter alia, the provided model, which has been trained by using real positions of real guidance devices that have been utilized during real ablation procedures, the virtual position of the virtual guidance device, which is used during the planning, can be more realistic.

The determination apparatus can also comprise a planning unit configured to plan an ablation procedure for ablating the ablation target based on the determined virtual position of the virtual guidance device and the provided image of the ablation target, and/or the determination apparatus can be configured to send the determined virtual position of the virtual guidance device to another apparatus which carries out the planning. Since the planning is based on a virtual position of a virtual guidance device, which is more likely realistically achievable during a real ablation procedure, the planning of the ablation procedure can be improved.

Generally, it would also be possible that the virtual guidance device is positioned manually during pre-planning, but such a manual positioning is often very crude. Moreover, repositioning a virtual guidance device manually in three dimensions is rather complex, wherein for this reason it might be very difficult or even impossible for a user to achieve a realistic position for the virtual guidance device. Also this can lead to an inaccurate planning. In contrast to this, by determining the virtual position of the virtual guidance device based on the provided image of the ablation target and the realistically trained model, the virtual position of the virtual guidance device corresponds much better to a realistic position, which can be achieved during the actual ablation procedure, thereby allowing for the improved planning.

Preferentially the model providing unit is configured to receive the training data sets from a device which is configured to be used during real ablation procedures and which knows a) real positions of real guidance devices that have been used during the real ablation procedures for ablating ablation targets and b) images of the ablation targets and/or parameters extracted from the images of the ablation target. This device can be, for instance, an ablation apparatus configured to carry out an ablation procedure. However, it can also be another device to be used during a real ablation procedure like an intermediate planning device which allows a user to adapt an initially planned ablation procedure based on an intermediate state and result of an ablation procedure and which therefore also knows a) real positions of real guidance devices that have been used during the real ablation procedures for ablating ablation targets and b) images of the ablation targets and/or parameters extracted from the images of the ablation target. For instance, it can be the UroNav device of the company Philips or a corresponding device of another company.

It is noted that a respective position preferentially defines a location and an orientation of the guidance device. The ablation target is preferentially a tumor and the image is preferentially an image which shows the ablation target and the surrounding of the ablation target. In particular, the image can show an organ of the subject, in which the ablation target is located. The image preferentially is a three-dimensional image. The image can be, for instance, a CT image, an MR image, an ultrasound image or an image acquired by using another imaging modality.

The image providing unit can be a storage in which the image is stored and from which the image can be retrieved for providing the same. The image providing unit can also be a receiving unit for receiving the image from another device like an imaging system and for providing the received image. The image providing unit can also be or comprise the imaging system itself, wherein the imaging system is configured to acquire the image. The image preferentially is a pre-operative image and can be, for instance, a radiology image like a CT image, an MR image, an ultrasound image or an image acquired by using another imaging modality.

The applicator is preferentially needle-like, in order to allow for a percutaneous ablation procedure. In a preferred embodiment, the applicator is configured to allow for a thermal ablation procedure. The applicator can be configured to provide, for instance, RF energy, MW energy, HIFU energy, FLA energy, IRE energy or to be used for cryoablation. The guidance device is preferentially a device which holds and thereby guides the applicator while placing the applicator in or close to the ablation target. In a preferred embodiment, the guidance device is a transperineal grid template. However, it can also be another type of guidance device.

In an embodiment the model providing unit is configured to provide a model to be trained by machine learning. The model can be, for instance, a neural network, particular a deep neural network. It has been found that by using a machine learning model like a neural network the virtual position of the virtual guidance device can be even better determined such that it corresponds to a realistically achievable position of a real guidance device.

Preferentially, the parameter extracted from the image defines a spatial location, wherein the model providing unit is configured to provide the model such that it is adapted to output the virtual position of the virtual guidance device relative to the spatial location defined by the parameter, if the model is provided, as an input, with the parameter. In particular, the parameter extracted from the image is at least one of a) a center of the ablation target, b) a center of an organ if the ablation target is located in the organ, c) a position of a fiducial marker if a fiducial marker is visible in the image and d) a predefined location relative to an outer box delimiting the image. It has been found that using these specific parameters further improves the quality of determining the virtual position of the virtual guidance device such that it even better corresponds to a realistically achievable position of a real guidance device.

The model providing unit preferentially is configured to train the model by averaging the training data sets. Preferentially, the averaging is an arithmetical averaging, a mean averaging or a mode averaging. The training preferentially is carried out accordingly. In an embodiment the real positions of the real guidance devices relative to the respective spatial locations defined by the respective parameters are averaged, in order to determine the model. For instance, corresponding vectors connecting a) a predefined location on the respective real guidance device in the respective real position and b) the respective spatial location defined by the respective parameter and having a predefined orientation relative to the real guidance device can be averaged for defining the model. It has been found that also by using this statistical model the determination of the virtual position of the virtual guidance device can be further improved such that it even better corresponds to a realistically achievable position of a real guidance device.

In an embodiment the model providing unit is configured to use a robust estimator to average the training data sets. For instance, the averaging of the training data sets can consider only a percentile like the 90^{th} percentile of all relative real positions of the real guidance devices of the training data sets. This can prevent that outliers adversely affect the determination of the virtual position of the virtual guidance device, thereby allowing for a further improved determination of this virtual position.

In an embodiment the model providing unit is configured to automatically receive from the device a new training data set including a) a new real position of the real guidance device that has been used during a new real ablation procedure for ablating an ablation target and b) a new image of the ablation target and/or a new parameter extracted from the new image, after the new real ablation procedure has been carried out with the new real position of the real guidance device and the new image of the ablation target. Moreover, in an embodiment the determination apparatus which determines the virtual position of the virtual guidance device and/or the device, which is, for instance, an ablation apparatus and which knows the real positions of the real guidance devices and corresponding images and/or parameters extracted from the images, comprises a user interface allowing a user to select which training data sets should be received from the device.

In an embodiment the determination apparatus further comprises a) a user interface configured to allow a user to modify the determined virtual position of the virtual guidance device, b) an allowable range determination unit configured to determine an allowable range of virtual positions of the virtual guidance device based on the training data sets, and c) an out of range determination unit configured to determine whether the modified determined virtual position of the virtual guidance device is out of the determined allowable range. Thus, the determined virtual position of the virtual guidance device can be provided as a start position, wherein, starting from this start position, the user can modify the virtual position as appropriate for the ablation procedure to be carried out. Since the user does not start from scratch when placing the virtual guidance device, the user is assisted in placing the virtual guidance device in a way which allows him/her to achieve a realistic position of the virtual guidance device, i.e. a position which is really achievable by a real guidance device during the actual ablation procedure.

In particular, the user can be warned, if the user has modified the virtual position of the virtual guidance device such that it is not anymore in an allowable range of positions. The allowable range determination unit can be configured to determine the allowable range of virtual positions of the virtual guidance device such that the virtual positions within the range correspond to positions which are really achievable during the actual ablation procedure. Thus, it can be ensured that still the virtual position of the virtual guidance device is really achievable during an actual ablation procedure, although the user is allowed to modify the initially determined virtual position. This allows for a further improved planning.

The allowable range of virtual positions can be defined by, for instance, the above described statistics. For instance, it can be defined by the standard deviation or another deviation measure being indicative for a deviation from the average of the real positions of the real guidance devices relative to the respective spatial location defined by the respective parameter.

In an embodiment the virtual position determination unit is configured to determine a multiplicity of virtual positions of the virtual guidance device based on the provided image of the ablation target and the provided model, wherein the planning apparatus further comprises a user interface configured to allow a user to select a virtual position from the determined multiplicity of virtual positions of the virtual guidance device.

Moreover, in an embodiment the model providing unit is configured to provide the model such that is adapted to output the virtual position of the virtual guidance device relative to the ablation target, if the model is provided, as the input, also with a covariate, wherein the training data sets, which are used for training the model, also include a respective covariate. Preferentially, the covariate is at least one of a group consisting of volume of the ablation target, volume of an organ comprising the ablation target, weight of the subject, amount of subcutaneous fat at an insertion site of an applicator, type of applicator and type of guidance device. By considering also at least one of these covariates, the quality of determining the virtual position of the virtual guidance device can be even further improved, which allows for a further improved planning of a subsequent ablation procedure.

In a further aspect a determination method for determining a virtual position of a virtual guidance device for guiding a virtual ablation applicator is presented, wherein the determination method comprises:
- providing a model that is adapted to output a virtual position of a virtual guidance device, if the model is provided, as an input, with an image of an ablation target or with a parameter extracted from the image of the ablation target, by a model providing unit, wherein the model providing unit trains the model based on training data sets, wherein each training data set includes a) a real position of a real guidance device that has been used for guiding a real ablation applicator during a real ablation procedure for ablating a respective ablation target and b) an image of the respective ablation target and/or a parameter extracted from the image,
- providing an image of the ablation target by an image providing unit, and
- determining the virtual position of the virtual guidance device based on the provided image of the ablation target and the provided model by a virtual position determination unit.

In another aspect a determination computer program for determining a virtual position of a virtual guidance device for guiding a virtual ablation applicator is presented, wherein the determination computer program comprises program code means for causing a determination apparatus as defined by any of claims 1 to 13 to carry out the steps of the determination method as defined by claim 14, when the computer program is run on the determination apparatus.

It shall be understood that the determination apparatus of claim 1, the determination method of claim 14 and the determination computer program of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily an embodiment of a determination apparatus for determining a virtual position of a virtual guidance device for guiding a virtual ablation applicator, wherein the determination apparatus receives training data sets from an ablation apparatus for ablating an ablation target,
Fig. 2 illustrates schematically and exemplarily parts of the ablation apparatus,
Fig. 3 illustrates schematically and exemplarily a view to be shown on a display of the determination apparatus, wherein the view includes a virtual guidance device, a virtual ablation applicator guided by the guidance device and a tumor to be ablated within an organ, and
Fig. 4 shows a flowchart exemplarily illustrating an embodiment of a determination method for determining a virtual position of a virtual guidance device for guiding a virtual applicator.

### DETAILED DESCRIPTION OF EMBODIMENTS

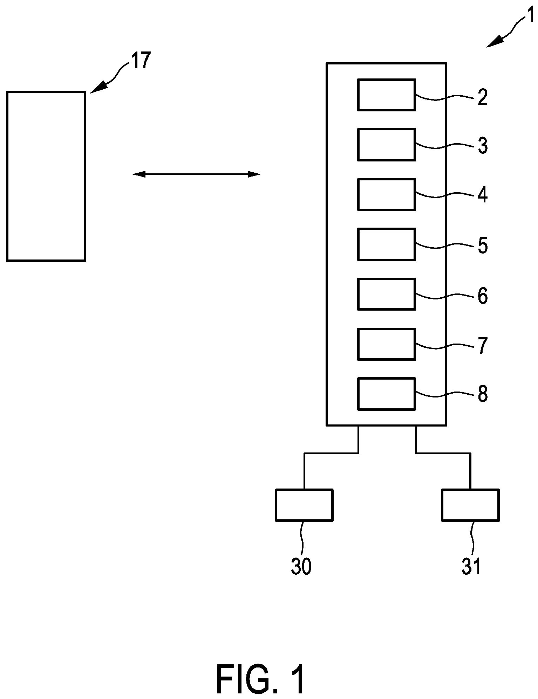

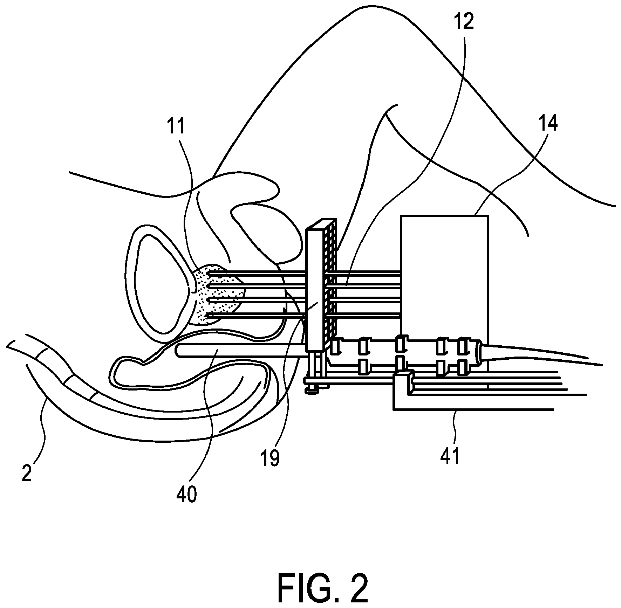

Fig. 1 shows schematically and exemplarily an embodiment of a determination apparatus 1 for determining a virtual position of a virtual guidance device for guiding a virtual ablation applicator. In this embodiment the determination apparatus 1 comprises a planning unit 8 configured to plan an ablation procedure for ablating an ablation region based on the determined virtual position of the virtual guidance device and a provided image of the ablation target. The determination apparatus 1 therefore can also be regarded as being a planning apparatus for planning an ablation procedure for ablating an ablation target within a subject. Moreover, in this embodiment the ablation procedure is a percutaneous thermal ablation procedure which will be described in the following with reference to Fig. 2.

The percutaneous thermal ablation procedure uses a transperineal grid template 19 as guidance device for guiding needle-like applicators 12 at or close to an ablation target being a tumor within the prostate 11 of a subject 21 in this embodiment. The applicators 12 are connected to an applicator control unit 14 which can be used to control the placement of the applicators 12 and the application of the ablation energy by the applicators 12.

During the percutaneous thermal ablation procedure ultrasound images are acquired by using an ultrasound probe 40. In this embodiment the ultrasound probe 40 is a transrectal ultrasound (TRUS) probe. The TRUS probe 40 can be, for instance, a tracked two-dimensional TRUS, which is used to acquire a three-dimensional volume by reconstruction from a series of two-dimensional images. It is also possible to directly use the two-dimensional TRUS images, i.e. without reconstructing a three-dimensional image. Generally, the TRUS probe can be configured to provide two-dimensional and/or three-dimensional images. The TRUS probe 40, the transperineal grid template 19 and the applicator control unit 14 are held by a holding element 41.

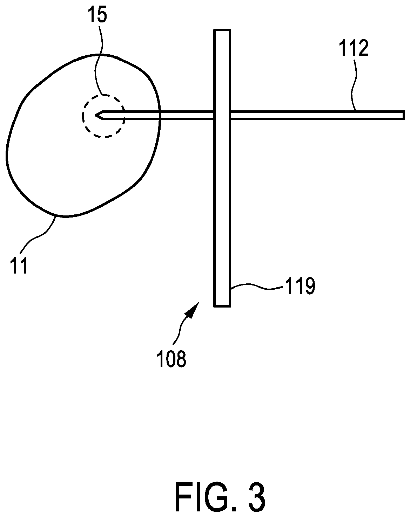

Referring again to Fig. 1, the planning apparatus 1 comprises a model providing unit 2 configured to provide a model that is adapted to output a virtual position of a virtual guidance device 119 relative to the ablation target 15, if the model is provided, as an input, with an image of the ablation target 15 or with a parameter extracted from the image of the ablation target 15. The virtual guidance device 119 is a virtual transperineal grid template configured to guide one or several virtual applicators 112 as schematically and exemplarily illustrated in Fig. 3. The virtual applicator 112 is configured to virtually apply ablation energy to the ablation target 15 being a tumor within the prostate 11. The model providing unit 2 is configured to train the model based on training data sets, wherein each training data set includes a) a real position of the real guidance device 19 and b) an image of an ablation target and/or a parameter extracted from the image. The planning apparatus 1 also comprises an image providing unit 3 configured to provide an image of the ablation target 15. In this embodiment the image is a pre-operative image like a CT or MR image. It is a three-dimensional image which shows the prostate 11 with the tumor 15.

The planning apparatus 1 further comprises a virtual position determination unit 4 configured to determine the virtual position of the virtual guidance device 119 based on the image provided by the image providing unit 3 and the model provided by the model providing unit 2. For instance, the virtual position determination unit 4 can be configured to extract a parameter from the provided image and to determine the virtual position of the virtual guidance device based on the extracted parameter and the model. The determined virtual position can then be shown on a display 31. In this embodiment, also a representation of the prostate 11 and the tumor 15 and the virtual applicator 112 are shown on the display 31 as illustrated in Fig. 3. Thus, the planning apparatus 1 can be configured to generate a view of the determined virtual position of the virtual guidance device 119, the virtual applicator 112, the prostate 11 and the tumor 15 and show the generated view on the display 31. The representation of the prostate 11 and the tumor 15 can be generated based on the provided image by using known techniques like segmentation

In an embodiment the parameter extracted from the image defines a spatial location, wherein the model providing unit 2 is configured to provide the model such that it is adapted to output the virtual position of the virtual guidance device relative to the spatial location defined by the parameter, if the model is provided, as an input, with the parameter. In particular, the parameter extracted from the image is at least one of a) a center of the ablation target 15, b) a center of the prostate 11, c) a position of a fiducial marker if a fiducial marker is visible in the image and d) a predefined location relative to an outer box delimiting the image. This predefined location can be, for instance, the center of the outer box or a corner of the outer box.

Moreover, the model providing unit 2 preferentially is configured to train the model by averaging the training data sets. For instance, an arithmetical averaging, a mean averaging or a mode averaging can be carried out. In an embodiment the real positions of the real guidance devices relative to the respective spatial locations defined by the respective parameters are averaged, in order to determine the model. For instance, corresponding vectors connecting a) a predefined location on the respective real guidance device in the respective real position and b) the respective spatial location defined by the respective parameter and having a predefined orientation relative to the real guidance device can be averaged for defining the model.

Moreover, in this embodiment the planning apparatus 1, in particular the model providing unit 2, is configured to receive the training data sets from an ablation apparatus 17 configured to carry out the ablation procedure such that the training data sets really include a) real positions of the real guidance device 19, which have been really used during real ablation procedures, and b) images of ablation targets and/or parameters extracted from the images. The model providing unit 2 can be configured to automatically receive from the ablation apparatus 17 a new training data set including a) a new real position of the real guidance device 19 and b) a new image of an ablation target and/or a new parameter extracted from the new image, after a new real ablation procedure has been carried out with the new real position of the real guidance device 19. It is also possible that the planning apparatus is configured to allow the user via the user interface 5 to select which training data sets should be received from the ablation apparatus 17. It is also possible that such a user interface for selecting the training data sets to be transmitted to the planning apparatus 1 is located on the ablation apparatus 17.

The user interface 5 or another user interface of the planning apparatus 1 can be configured to allow a user to modify the determined virtual position of the virtual guidance device 119 by using input means 30 like a keyboard, a computer mouse, a touch screen, et cetera. Moreover, the planning apparatus 1 comprises a) an allowable range determination unit 6 configured to determine an allowable range of virtual positions of the virtual guidance device 119 based on the training data sets and b) an out of range determination unit 7 configured to determine whether the modified determined virtual position of the virtual guidance device 119 is out of the determined allowable range. Thus, starting from the initial virtual position of the virtual guidance device 119, which has been determined by the virtual position determination unit 4, the user can modify the virtual position of the virtual guidance device 119, wherein, if the modified determined virtual position of the virtual guidance device 119 is out of a range of virtual positions which are achievable by the real guidance device 19, this can be detected by the out of range determination unit 7 and a corresponding warning can be provided to the user via the display 31 or via another output unit. The warning can be an acoustical warning and/or an optical warning. The allowable range determination unit 6 can be configured to determine the allowable range of virtual positions of the virtual guidance device such that it corresponds to the real positions of the real guidance device of the training data sets. In particular, the allowable range determination unit can be configured such that the user is warned if the modified, i.e. the manually adjusted, positions fall outside of a statistical norm. For instance, the statistical norm and hence the allowable range can be defined by the standard deviation of an average of the real positions of the real guidance device relative to respective spatial locations defined by the respective parameters extracted from the respective images, wherein the determined standard deviation or another deviation measure can define an allowable range.

In an embodiment, the real positions of the real guidance device are presented as quaternion representations, wherein an eigendecomposition is applied to these quaternion representations. The resulting first eigenvector represents the average position and the further eigenvectors represent the principle components of variation that can be scaled to calculate the allowable range of virtual positions of the virtual guidance device. However, it is also possible to, as indicated above, average vector representations of the real positions of the real guidance device relative to the respective spatial location defined by the respective parameter to calculate an average vector position and to also calculate a standard deviation vector position, wherein the standard deviation vector position defines the allowable range of virtual positions.

The model providing unit 2 can be configured to provide the model such that it is adapted to output the virtual position of the virtual guidance device 119 relative to the ablation target 15, if the model is provided, as the input, also with a covariate, wherein the model providing unit 2 is configured to train the model based on the training data sets, wherein each training data set also includes a respective covariate. The covariate can be at least one of a group consisting of volume of the ablation target 15, volume of the prostate 11, weight of the subject 21, amount of subcutaneous fat at an insertion site of an applicator, type of applicator and type of guidance device.

The model can be any means which provides a relation between a) an image of an ablation target or a parameter extracted from the image of the ablation target and b) a virtual position of a virtual guidance device, wherein this means is trainable by using the training data sets including at least real positions of real guidance devices and real images of real ablation targets and/or parameters extracted from the real images and optionally also covariates. The means, i.e. the model, can be a machine learning model like a neural network, in particular a deep neural network, or also a statistical model as explained above. The deep neural network is particularly used, if non-numerical covariates are applied like guidance device type, scan protocol, et cetera. The covariates will be explained in more detail further below.

The planning apparatus 1 comprises the planning unit 8 configured to plan an ablation procedure for ablating the ablation region based on the determined and optionally modified virtual position of the virtual guidance device 119 and the provided image of the ablation target 15. In particular, the planning unit 8 can be adapted to determine the parameters of an ablation plan automatically based on the pre-operative image, the ablation target indicated in the pre-operative image and surrounding organs also indicated in the pre-operative image and also based on a prescription how much ablation energy should be received by which part. For this determination of the parameters of the ablation plan known ablation planning algorithms can be used. The planning unit 8 can be further adapted to allow the user to modify the ablation plan, i.e. to modify the parameters of the ablation plan and to simulate the ablation procedure based on the amended parameters. The simulation can be shown to the user, wherein, if the user is satisfied with the simulation, the ablation plan with the possibly amended parameters can be transmitted to the ablation apparatus 17. The parameters of the ablation plan can include, for instance, the amount of ablation energy to be applied by a respective applicator, the time period over which the respective amount of ablation energy should be applied, the position of the respective applicator which should apply the ablation energy, et cetera.

The ablation apparatus 17 can comprise the applicator control unit 14 described above with reference to Fig. 2. The applicator control unit 14 can comprise a user interface allowing a user to set control parameters like the ablation energy or the time period of applying the ablation energy to be used during the ablation procedure. The user can set these control parameters based on an ablation plan generated by the planning unit 8. The user also can manually place the applicators 12 in accordance with the ablation plan. However, it is also possible that the applicator control unit 14 allows to automatically or semi-automatically control the applicators 12 and hence the ablation procedure based on the ablation plan generated by the planning unit 8. In an embodiment also the ablation apparatus comprises a planning unit, in order to allow the ablation apparatus, i.e. the planning unit of the ablation operators, to adapt the initial plan generated by the planning unit 8 of the planning apparatus 1 based on, for instance, an intermediate state present during the actual ablation procedure.

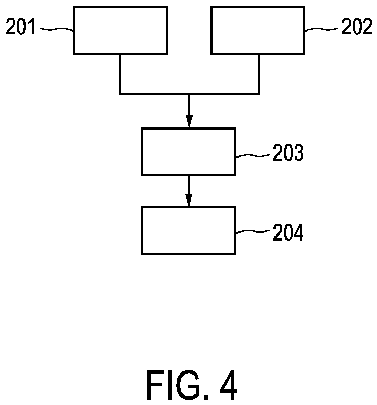

In the following, an embodiment of a determination method for determining a virtual position of a virtual guidance device for guiding a virtual applicator, which in this embodiment comprises a planning step and therefore could also be regarded as being a planning method for planning an ablation procedure for ablating an ablation target within a subject, will exemplarily be described with reference to a flowchart shown in Fig. 4.

In step 201, a model is provided, which is adapted to output a virtual position of a virtual guidance device, if the model is provided, as an input, with an image of the ablation target or with a parameter extracted from the image of the ablation target by a model providing unit. The virtual guidance device is configured to guide a virtual applicator which is adapted to virtually apply ablation energy to the ablation target, wherein the model providing unit 2 is configured to train the model based on training data sets. Each training data set includes a) a real position of a real guidance device and b) an image of an ablation target and/or a parameter extracted from the image. In step 202, an image of the ablation target is provided by the image providing unit 3 and, in step 203, the virtual position of the virtual guidance device is determined based on the provided image of the ablation target and the provided model by the virtual position determination unit 4. In step 204, an ablation procedure for ablating the ablation target 15 is planned based on the determined virtual position of the virtual guidance device and the provided image of the ablation target by the planning unit 8.

The determination apparatus and the determination method can be used in interventional oncology, but also in other medical fields in which an ablation target should be ablated. In particular, the determination apparatus and the determination method allow for an accurate virtual positioning of guidance devices in pre-planning scenarios. The virtual position of guidance devices like the transperineal grid template can be estimated, i.e. determined, based on prior uses of these devices during actual interventional procedures, i.e. during actual ablation procedures. The determination apparatus and determination method can rely on a communication between the determination apparatus, which in an embodiment could also be regarded as being a pre-planning apparatus, and the ablation apparatus preferentially being a fusion-guided interventional system for the delivery of the ablative therapy. In particular, the ablation apparatus can be configured to fuse an interventional image, i.e. an image which has been acquired during the interventional ablation procedure, and a pre-interventional or pre-operative image and show the resulting fused image on a display to a user, in order to guide the user during the ablation procedure. Due to the communication between the ablation apparatus and the determination apparatus, the determination apparatus can receive the pre-interventional or pre-operative images and also the respectively used position of the guidance devices like the position of the transperineal grid template as training data sets for training the model.

If the determination apparatus comprises the planning unit and hence could be regarded as being a planning apparatus, the ablation plan determined by using the planning apparatus can be sent to the ablation apparatus, wherein the ablation procedure then is carried out in accordance with the ablation plan. The ablation plan comprises, for instance, positions of one or several applicators, positions of one or several guidance devices, the amount of ablation energy to be applied at the respective position, the type of ablation energy, et cetera. During the ablation procedure, the user might deviate from the planned parameters, wherein the actually used parameters like the actually used position of the one or more guidance devices can be transmitted from the ablation apparatus to the planning apparatus together with further information like the respective pre-operative radiology image. This information received for several ablation procedures can form the training data sets used by the planning apparatus for training the model.

The ablation apparatus knows which finally used real position of a real guidance device belongs to which pre-operative image, i.e. in particular to which pre-operative radiology image, because the ablation apparatus knows which pre-operative image and which real position of the real guidance device has been used in which respective ablation procedure. Due to being used in a same respective ablation procedure, a respective pre-operative image and a respective real position of the real guidance device are linked and form a training data set which is sent from the ablation apparatus to the determination apparatus. The training data sets then allow the determination apparatus to learn how to position the one or more guidance devices in future ablation procedures.

In an embodiment, the real guidance device and the TRUS probe 40 are tracked by using known tracking technology like electromagnetic or optical tracking, wherein this tracking technology can be carried out by the ablation apparatus for determining the current position of the real guidance device relative to a TRUS image acquired by using the TRUS probe 40. Moreover, in this embodiment, the ablation apparatus is configured to register the pre-operative image with the TRUS image. A concatenation of an image registration transformation and a tracking transformation then provides a link between the pre-operative image and the real position of the guiding device, wherein by using this link a training data set can be formed which can be sent from the ablation apparatus to the determination apparatus.

The determination apparatus having access to the stored training data sets created by the ablation apparatus can propose a virtual position of the guidance device by using one or more parameters, in particular one or more statistics, including the mean center of the scan, the mean center of the ablation target, the mean center of the organ in which the target is located like the mean center of the prostate particularly for focal ablation procedures and a mean center of a fiducial marker visible in the pre-operative images. For instance, if a fiducial marker is placed inside, or onto, a patient ahead of pre-operative imaging in a standardized way, the virtual guidance device position can be expressed by the mean position with respect to the center of this fiducial marker. The mean could be, for instance, the arithmetic mean, the median or the mode. Moreover, a robust estimation can be used like calculating the mean of all training data sets within a certain percentile like a 90^{th} percentile.

As explained above, the determination apparatus can comprise a user interface, particularly a graphical user interface, in order to allow the user to upload only selected historical cases, i.e. only selected training data sets, to the determination apparatus. In addition or alternatively, such a user interface might also be provided on the ablation apparatus. Moreover, as also explained above, it is possible that an automatic service is present, wherein historical data, i.e. new training data sets, are updated and hence transferred from the ablation apparatus to the determination apparatus as soon as new cases are available on the ablation apparatus. The addition of new samples, i.e. the addition of new training data sets, can lead to a different initial positioning of the virtual guidance device as determined by the virtual position determination unit 4. In particular, starting from an empty database, the determination apparatus can gradually become smarter, as the number of cases to base its estimates, i.e. the number of training data sets, increases.

The determination apparatus can be adapted to propose a multiplicity of different initial positionings for the applicator guidance device. Thus, the virtual position determination unit 4 can be configured to determine a multiplicity of virtual positions of the virtual guidance device based on the provided image of the ablation target and the provided model. The user interface of the determination apparatus then can be further configured to allow a user to select a virtual position from the determined multiplicity of virtual positions of the virtual guidance device.

In addition to collecting, for instance, the actual grid positioning during the ablation procedures, also various covariates can be collected and transmitted to the determination apparatus as explained above. Using these covariates for determining the virtual position of the virtual guidance device can help to improve this virtual positioning. For instance, as patient-to-patient variations may require the guidance device to be positioned differently, considering anatomical characteristics as covariates can allow to adapt the virtual positioning of the guidance device to the respective patients. Covariates that may be used to achieve this include the target volume, the volume of an organ containing the target, the weight of the patient and the amount of subcutaneous fat at an insertion site.

The real physical positioning of the guidance device can also depend on the devices which are used during the actual ablation procedure. Thus, also the virtual position of the virtual guidance device can depend on these parameters such that also, for instance, the applicator type, the guidance device type or a stepper type, which might be used in a prostate procedure, can be used as covariates.

The determination of the virtual position of the virtual guidance device based on the covariates can be implemented using traditional statistics, but it is also particularly suitable to be implemented by using artificial intelligence techniques like by using deep neural networks. If the model uses a deep neural network, the respective covariate can be a further input to the deep neural network to be trained.

The training data sets can be grouped with respect to the covariates and then the respective group can be used for training a respective model such that for each group and hence for each covariate value or range of covariate values there is a certain trained model. For instance, if the patient weight is considered as a covariate, for example, three models might be provided including a first model for weights smaller than 60 kg, a second model for weights between 60 kg and 80 kg and a third model for weights being larger than 80 kg.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the training of the model, the determination of the virtual position of the virtual guidance device and the planning of the ablation procedure performed by one or several units or devices can also be performed by any other number of units or devices. These procedures and/or the control of the determination apparatus in accordance with the determination method can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a determination apparatus for determining a virtual position of a virtual guidance device like a virtual transperineal grid template for guiding a virtual ablation applicator like a virtual ablation needle. A model is trained based on training data sets including real positions of real guidance devices used for guiding real ablation applicators during real ablation procedures and images of ablation targets and/or parameters extracted from the images. The parameters are, for instance, spatial parameters like the center of the respective ablation target. The model then is used for determining the virtual position of the virtual guidance device based on an image of the ablation target, which might be a pre-operative CT or MR image, and the model. This allows to provide a virtual position, which really is achievable by a real guidance device, wherein this allows for an improved ablation planning.

## Claims

1. A determination apparatus for determining a virtual position of a virtual guidance device (119) for guiding a virtual ablation applicator (112), wherein the determination apparatus (1) comprises:
- a model providing unit (2) configured to provide a model that is adapted to output a virtual position of a virtual guidance device (119), if the model is provided, as an input, with an image of an ablation target (15) or with a parameter extracted from the image of the ablation target (15), wherein the model providing unit (2) is configured to train the model based on training data sets, wherein each training data set includes a) a real position of a real guidance device (19) that has been used for guiding a real ablation applicator during a real ablation procedure for ablating a respective ablation target and b) an image of the respective ablation target and/or a parameter extracted from the image,
- an image providing unit (3) configured to provide an image of the ablation target (15), and
- a virtual position determination unit (4) configured to determine the virtual position of the virtual guidance device (119) based on the provided image of the ablation target (15) and the provided model.

2. The determination apparatus as defined by claim 1, wherein the parameter extracted from the image defines a spatial location, wherein the model providing unit (2) is configured to provide the model such that it is adapted to output the virtual position of the virtual guidance device (119) relative to the spatial location defined by the parameter, if the model is provided, as an input, with the parameter.

3. The determination apparatus as defined by claim 2, wherein the parameter extracted from the image is at least one of a) a center of the ablation target, b) a center of an organ (11) if the ablation target is located in the organ (11), c) a position of a fiducial marker if a fiducial marker is visible in the image and d) a predefined location relative to an outer box delimiting the image.

4. The determination apparatus as defined by any of the preceding claims, wherein the model providing unit (2) is configured to train the model by averaging the training data sets.

5. The determination apparatus as defined by claim 4, wherein the model providing unit (2) is configured to use a robust estimator to average the training data sets.

6. The determination apparatus as defined by any of the preceding claims, wherein the model providing unit (2) is configured to receive the training data sets from a device (17) which is configured to be used during real ablation procedures and which knows a) real positions of real guidance devices (19) that have been used during the real ablation procedures for ablating ablation targets and b) images of the ablation targets and/or parameters extracted from the images of the ablation targets.

7. The determination apparatus as defined by claim 6, wherein the model providing unit (2) is configured to automatically receive from the device (17) a new training data set including a) a new real position of the real guidance device (19) that has been used during a new real ablation procedure for ablating an ablation target and b) a new image of the ablation target (15) and/or a new parameter extracted from the new image, after the new real ablation procedure has been carried out with the new real position of the real guidance device (19).

8. The determination apparatus as defined by any of claims 6 and 7, wherein the determination apparatus (2) comprises a user interface (5) allowing a user to select which training data sets should be received from the device (17).

9. The determination apparatus as defined by any of the preceding claims, wherein the determination apparatus (1) further comprises:
- a user interface (5) configured to allow a user to modify the determined virtual position of the virtual guidance device (119),
- an allowable range determination unit (6) configured to determine an allowable range of virtual positions of the virtual guidance device (119) based on the training data sets, and
- an out of range determination unit (7) configured to determine whether the modified determined virtual position of the virtual guidance device (119) is out of the determined allowable range.

10. The determination apparatus as defined by any of the preceding claims, wherein the model providing unit (2) is configured to provide the model such that it is adapted to output the virtual position of the virtual guidance device (119), if the model is provided, as the input, also with a covariate, wherein the training data sets, which are used for training the model, also include a respective covariate.

11. The determination apparatus as defined by claim 10, wherein the covariate is at least one of a group consisting of volume of the ablation target (15), volume of an organ (11) comprising the ablation target (15), weight of the subject (21), amount of subcutaneous fat at an insertion site of an applicator, type of applicator and type of guidance device.

12. The determination apparatus as defined by any of the preceding claims, wherein the model providing unit (2) is configured to provide a model to be trained by machine learning as the model.

13. The determination apparatus as defined by any of the preceding claims, wherein the apparatus further comprises a planning unit (8) configured to plan an ablation procedure for ablating the ablation target (15) based on the determined virtual position of the virtual guidance device (119) and the provided image of the ablation target (15).

14. A determination method for determining a virtual position of a virtual guidance device (119) for guiding a virtual applicator (112), wherein the determination method comprises:
- providing a model that is adapted to output a virtual position of a virtual guidance device (119), if the model is provided, as an input, with an image of an ablation target (15) or with a parameter extracted from the image of the ablation target (15) by a model providing unit (2), wherein the model providing unit (2) trains the model based on training data sets, wherein each training data set includes a) a real position of a real guidance device (19) that has been used for guiding a real ablation applicator during a real ablation procedure for ablating a respective ablation target and b) an image of the respective ablation target and/or a parameter extracted from the image,
- providing an image of the ablation target (15) by an image providing unit (3), and
- determining the virtual position of the virtual guidance device (119) based on the provided image of the ablation target (15) and the provided model by a virtual position determination unit (4).

15. A determination computer program for determining a virtual position of a virtual guidance device (119) for guiding a virtual ablation applicator (112), the determination computer program comprising program code means for causing a determination apparatus as defined by any of claims 1 to 13 to carry out the steps of the determination method as defined by claim 14, when the determination computer program is run on the determination apparatus.
